(19)

**Europäisches Patentamt**
**European Patent Office**
**Office européen des brevets**

(11)　**EP 3 028 190 B1**

(12)　　　　**EUROPEAN PATENT SPECIFICATION**

(45) Date of publication and mention
of the grant of the patent:
**22.06.2022 Bulletin 2022/25**

(21) Application number: **14832137.5**

(22) Date of filing: **31.07.2014**

(51) International Patent Classification (IPC):
**G16H 20/40** *(2018.01)*　　**G16H 50/20** *(2018.01)*
**G16H 50/70** *(2018.01)*　　**G16H 10/60** *(2018.01)*
**G06F 40/20** *(2020.01)*

(52) Cooperative Patent Classification (CPC):
**G06F 40/20; G16H 10/60; G16H 20/40;**
**G16H 50/20; G16H 50/70**

(86) International application number:
**PCT/US2014/049301**

(87) International publication number:
**WO 2015/017731 (05.02.2015 Gazette 2015/05)**

(54) **IDENTIFICATION OF SURGERY CANDIDATES USING NATURAL LANGUAGE PROCESSING**

IDENTIFIKATION VON CHIRURGIEKANDIDATEN DURCH NATÜRLICHE
SPRACHVERARBEITUNG

IDENTIFICATION DE CANDIDATS À LA CHIRURGIE PAR TRAITEMENT DU LANGAGE NATUREL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **01.08.2013 US 201361861173 P**

(43) Date of publication of application:
**08.06.2016 Bulletin 2016/23**

(73) Proprietor: **Children's Hospital Medical Center
Cincinnati, OH 45229 (US)**

(72) Inventors:
 • **PESTIAN, John, P.**
**Cincinnati, OH 45229 (US)**
 • **GLAUSER, Tracy, A.**
**Cincinnati, OH 45229 (US)**
 • **HOLLAND, Katherine, D.**
**Cincinnati, OH 45229 (US)**
 • **STANDRIDGE, Shannon, Michelle**
**Cincinnati, OH 45229 (US)**
 • **GREINER, Hansel, M.**
**Cincinnati, OH 45229 (US)**
 • **COHEN, Kevin, Bretonnel**
**Denver, CO 80207 (US)**

(74) Representative: **Forresters IP LLP
Skygarden
Erika-Mann-Straße 11
80636 München (DE)**

(56) References cited:
**WO-A1-2010/126867　　WO-A1-2012/025765
US-A1- 2008 201 280　　US-A1- 2010 262 603
US-A1- 2012 041 911　　US-A1- 2013 035 961
US-A1- 2013 035 961**

 • **ZENG QING T ET AL: "Extracting principal
diagnosis, co-morbidity and smoking status for
asthma research: evaluation of a natural
language processing system", BMC MEDICAL
INFORMATICS AND DECISION MAKING, BIOMED
CENTRAL, LONDON, GB, vol. 6, no. 1, 26 July
2006 (2006-07-26), page 30, XP021017524, ISSN:
1472-6947, DOI: 10.1186/1472-6947-6-30**
 • **JUFFALI, W ET AL.: 'The WINAM Project: Neural
Data Analysis with Applications to Epilespy.'
BIOMEDICAL CIRCUITS AND SYSTEMS
CONFERENCE (BIOCAS 2010, pages 45 - 48,
XP031899695**

Description

FIELD OF THE INVENTION

The present invention relates to the use of natural language processing in systems and methods for clinical decision support.

BACKGROUND OF THE INVENTION

Epilepsy is a disease characterized by recurrent seizures that may cause irreversible brain damage. While there are no national registries, epidemiologists have shown that roughly three million Americans require $17.6 billion USD in care annually to treat their epilepsy. Epilepsy is defined by the occurrence of two or more unprovoked seizures in a year. Approximately 30% of those individuals with epilepsy will have seizures that do not respond to anti-epileptic drugs (Kwan et al., NEJ Med. (2000) 342(5):314-319). This population of individuals is said to have intractable or drug-resistant epilepsy (Kwan et al., Epilepsia (2010) 51(6): 1069-1077).

Select intractable epilepsy patients are candidates for a variety of neurosurgical procedures that ablate the portion of the brain known to cause the seizure. On average, the gap between the initial clinical visit when the diagnosis of epilepsy is made and surgery is six years. A need exists to predict which patients should be considered candidates for referral to surgery earlier in the course of treatment in order to mitigate the adverse effects on patients caused by years of damaging seizures, under-employment, and psychosocial distress. The present invention addresses this need by providing a method to identify patients having an intractable form of epilepsy. The methods of the invention utilize predictive models based upon the analysis of the clinical notes of epilepsy patients to identify patients likely to benefit from surgical intervention.

Although there has been extensive work on building predictive models of disease progression and of mortality risk, few models take advantage of natural language processing in addressing this task. One group used univariate analysis, multivariate logistic regression, sensitivity analyses, and Cox proportional hazards models to predict 30-day and 1-year survival of overweight and obese Intensive Care Unit patients. As one of the features in their system, they used smoking status extracted from patient records by natural language processing techniques. Himes et al. (J. Am. Med. Inform. Assoc. 16(3): 371-379 2009) used a Bayesian network model to predict which asthma patients would go on to develop chronic obstructive pulmonary disease. As one of their features, they also used smoking status extracted from patient records by natural language processing progression of time points were examined to gain insight into how the linguistic characteristics (and natural language processing-based classification performance) evolve over treatment course. Linguistic features that characterize the differences between the document sets from the two groups of patients were also studied.

It has been observed that 'the complexity of modern medicine exceeds the inherent limitations of the unaided human mind". *See e.g.*, Haug, P.J. J. Am. Med. Inform. Assoc. (2013) e102-e110. This complexity is reflected in the large amounts of data, both patient-specific and population based, available to the clinician. But the shear amount of information presents the clinician with substantial challenges such as focusing on the relevant information ('data'), aligning that information with standards of clinical practice ('knowledge'), and using that combination of data and knowledge to deliver care to patients that reflects the best available medical evidence at the time of treatment. *Id.*

The course of treatment for epilepsy follows two basic paths. Some patients respond to medical or other non-surgical interventions and are said to be "non-intractable." Other patients do not respond to medical or other non-surgical interventions. These patients are said to be "intractable." They are referred for consultation for surgical intervention, and may receive surgery if it is appropriate. Currently, from the time of the initial consultation to the time when a patient is referred for surgery is about 6 years. There is a need to identify patients who are candidates for surgery earlier than is currently possible. Earlier identification of such patients would improve patient quality of life and limit or reduce the long-term adverse effects of the seizures, whose damage to the brain is believed to be cumulative. The present invention addresses this need and helps patients with intractable seizures receive appropriate treatment faster.

US patent publication US2013/035961(A1) discloses techniques for applying user corrections to medical fact extraction including extracting a first set of one or more medical facts from a first portion of text documenting a patient encounter. A correction to the first set of medical facts may be received from a user. The correction may identify a fact that should be associated with the first portion of the text. A second set of one or more medical facts may be extracted from a second portion of the text based at least in part on the user's correction to the first set of medical facts. Extracting the second set of facts may include extracting one or more facts similar to the identified fact from the second portion of the text.

SUMMARY OF THE INVENTION

The examples, aspects and embodiments, if any, disclosed in the following description that do not fall within the scope of the claims are for reference only, and are to be interpreted as examples useful for understanding various embodiments of the invention. The systems and methods of the invention are based upon the inventors' discovery that epilepsy patients having intractable epilepsy, meaning they will fail to respond to non-surgical therapies and eventually be referred for surgery, and those having non-intractable epilepsy, meaning they do respond to non-surgical therapies, can be differentiated based upon clinical text from their medical records, specifically based on clinical text in the form of "free text". In this context, the term "free text" refers to the notes written by medical personnel in the patient's medical records. Advantageously, the methods of the invention can identify patients having intractable epilepsy, and who should therefore be referred for surgery, as much as two years before they would otherwise have been identified using traditional methods.

The present invention therefore relates to computer-based clinical decision support tools, including, computer-implemented methods, computer systems, and computer program products for clinical decision support. These tools assist the clinician in identifying epilepsy patients who are candidates for surgery and utilize a combination of natural language processing, corpus linguistics, and machine learning techniques. The present invention applies these techniques to identify patients who are candidates for surgery, thereby providing the clinician with a valuable tool for epilepsy care and treatment. The systems and methods of the invention identify an epilepsy patient as having intractable epilepsy, and therefore as a candidate for surgery, at least one or two years earlier than existing methods.

The invention provides a clinical decision support (CDS) tool for the identification of epilepsy patients who are candidates for surgery, the CDS tool comprising a non-transitory computer readable medium storing instructions that, when executed by at least one programmable processor, cause the at least one programmable processor to perform operations comprising: receiving, by a computing device, a set of data consisting of n-grams extracted from a corpus of clinical text of an epilepsy patient; classifying the data into one of two bins consisting of "intractable epilepsy" or "non-intractable epilepsy" by applying by a computer implemented method selected from a linquistic method and a machine learning method; and outputting the result,thereby providing clinical decision support for the identification of epilepsy patients who are candidates for surgery.

In one embodiment, the operations further comprise one or both of extracting the n-grams from the corpus of clinical text prior to or concurrent with receiving the set of data and structuring the data prior to classifying. The operation of structuring the data may include one or more of tagging parts of speech, replacing abbreviations with words, correcting misspelled words, converting all words to lower-case, and removing n-grams containing non-ASCII characters. The data may be further structured by removing words found in the National Library of Medicine stopwords list.

In one embodiment, the operations further comprise querying a database of electronic records to identify the clinical text for inclusion in the corpus.

The classifying step is performed by applying a classifier selected from a pre-trained support vector machine (SVM), a log-likelihood ratio, Bayes factor, or Kullback-Leibler Divergence. In one embodiment, the classifying step is performed by applying a pre-trained SVM

The classifier is trained on a training set comprising or consisting of two sets of n-grams extracted from two corpora of clinical text, a first corpus consisting of clinical text from a population of epilepsy patients that were referred for surgery and a second corpus consisting of clinical text from a population of epilepsy patients that were never referred for surgery. In one embodiment, each document of the corpora of clinical text satisfies each of the following criteria: it was created for an office visit, it is over 100 characters in length, it comprises an ICD-9-CM code for epilepsy, and it is signed by an attending clinician, resident, fellow, or nurse practitioner. In one embodiment, each patient of the population of patients is represented by at least 4 documents, each from a separate office visit.

In one embodiment, the set of data or training set is annotated with term classes and subclasses of an epilepsy ontology. The term classes may comprise one or more, or all, of the following: seizure type, etiology, epilepsy syndrome by age, epilepsy classification, treatment, and diagnostic testing. The annotating may be performed by human experts, or via a computer-implemented method, or by a combination of human and computerized methods.

In one embodiment, the n-grams are selected from one or more of unigrams, bigrams, and trigrams.

In one embodiment, the operations are performed at regular intervals. In one embodiment, the regular intervals are selected from daily, weekly, biweekly, monthly, and bimonthly.

In one embodiment, the patient is a pediatric patient.

In one embodiment, the result is displayed on a graphical user interface. The result may comprise one or a combination of two or more of text, color, imagery, or sound.

In one embodiment, the outputting operation further comprises sending an alert to an end-user if the results of the classification are "intractable" and the patient had a previous result of "non-intractable". In one embodiment, the alert is in the form of a visual or audio signal that is transmitted to a computing device selected from a personal computer, a tablet computer, and a smart phone. In one embodiment, the alert is manifested as any of an email, a text message, a voice message, or sound.

The invention also provides a method for the identification of epilepsy patients who are candidates for surgery, the method comprising use of the CDS tool described herein.

The invention also provides a system comprising the at least one programmable processor of the CDS tool described herein operatively linked to one or more databases of electronic medical records and/or clinical data. The at least one programmable processor can be coupled to a storage system, at least one input device, and at least one output device. The at least one programmable processor can receive data and instructions from, and can transmit data and instructions to, the storage system, the at least one input device, and the at least one output device. In one embodiment, the system comprises at least one of a back-end component, a middleware component, a front-end component, and one or more combinations thereof. The back-end component can be a data server. The middleware component can be an application server. The front-end component can be a client computer having a graphical user interface or a web browser, through which a user can interact. In one embodiment, the system comprises clients and servers. A client and server can be generally remote from each other and can interact through a communication network. The relationship of client and server can arise by virtue of computer programs running on the respective computers and having a client-server relationship with each other.

## BRIEF DESCRIPTION OF THE FIGURES

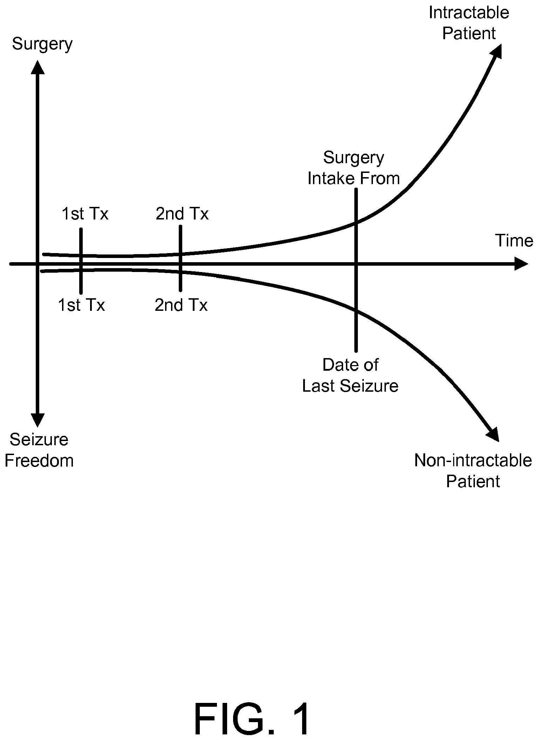

Figure 1: the two major paths in epilepsy care and treatment which ultimately divide the patient population into two groups, those having intractable epilepsy which does not respond to non-surgical therapies and non-intractable epilepsy, which does respond to non-surgical therapies.

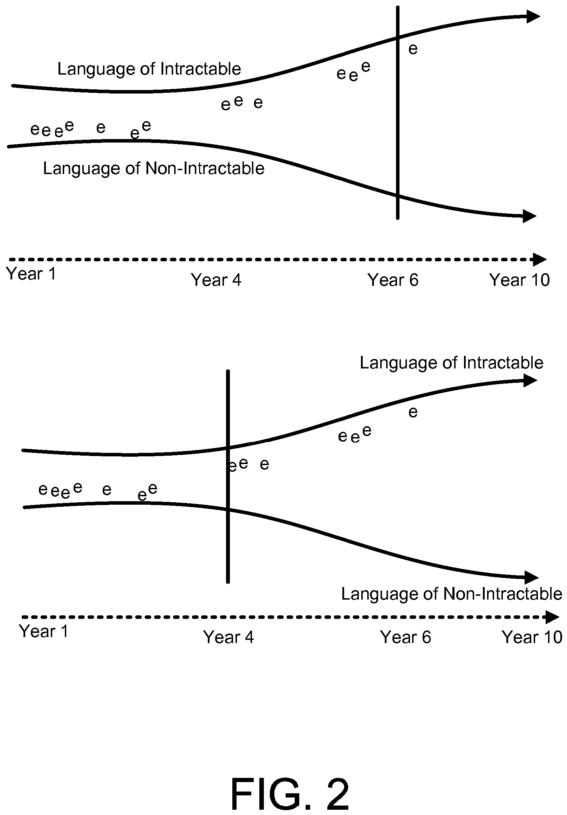

Figure 2: Graphical depiction of the advantages of the claimed methods in the identification of patients having intractable epilepsy. Top shows that the features of intractable and non-intractable language begin to diverge around year 4 and are noticeable by clinicians around year six. Bottom shows that the features begin to diverge around year 4 and are detectable by the methods of the invention at year four.

## DETAILED DESCRIPTION OF THE INVENTION

The invention provides tools for clinical decision support in the form of computer-implemented methods for identifying epilepsy patients who are candidates for surgery. Patients who are candidates for surgery may be referred to interchangeably herein as "intractable" patients, patients having intractable epilepsy, or patients who are candidates for referral to surgery. The methods utilize data extracted from the clinical notes of a patient to classify the patient into one of two groups, intractable or non-intractable. The clinical notes are in electronic form and may be accessed, for example, by querying a database or data warehouse of electronic medical records or clinical data. The data comprise or consist of "free text" from clinical documents, also referred to herein as "clinical free text". Typically, the clinical documents contain progress notes of the patient taken by a clinician who may be an attending physician, a resident, a fellow, or a nurse practitioner, over the course of at least 2, preferably at least 4 visits by the patient to a clinic or hospital. The data utilized for classification consists of n-grams in the form of words extracted from the clinical free text. The n-grams may be one or more of unigrams, bigrams, and trigrams. In one embodiment, the n-grams are in the form of words extracted from clinical documents and consist of unigrams or bigrams, or a combination thereof.

Data may be received into the system by direct input, for example by a user, or through querying an electronic record or a database of electronic records, including for example electronic health records (EHRs) or a warehouse of clinical data, *e.g.*, through a computer network linked to one or more databases of electronic records. The databases may include records from one or more clinics or hospitals. Data relevant to the classification of the patient as intractable or non-intractable may be identified and extracted, for example, by one or more tools of natural language processing using features of the data such as a unique patient identifier and ICD-9 codes, for example, ICD-9-CM codes for epilepsy. In one embodiment, data is extracted from EHRs contained within an electronic medical record system using a series of scripts, such as PL/SQL scripts.

The data may be received in either structured or unstructured form. Where the data is in unstructured form, the data is structured prior to classification. Structuring the data may include, for example, converting words to lower-case, substituting with the string NUMB if the n-gram is a numeral, removing n-grams that are either a non-ASCII character or a word found in the National Library of Medicine stopwords list.

Following data extraction and structuring, or upon receiving structured data, the system applies a classifier to bin the data into one of two bins, "intractable" or "non-intractable", and output the result of the classification. In one embodiment, the result may comprise a probability score or some indicator of the confidence level or strength of the classification. In one embodiment, the result is output visually in a manner that incorporates one or more of descriptive text, a color, or a symbol. In one embodiment, the result is output in a transmissible form such that they can be transmitted to a user, for example via email, SMS, or other similar technology. In one embodiment, the system is configured to alert a user if a patient's classification changes from non-intractable to intractable. The alert may be in the form of a visual or audio alert, and may also be in the form of an email, text message, or voicemail delivered to a user.

The classifier may utilize corpus linguistic methods or machine learning methods, or a combination of the two. In one embodiment, the classifier utilizes a methodology selected from an information-theoretic approach, a statistical approach, a machine learning approach, and a Bayesian approach. In one embodiment, the classifier utilizes a methodology selected from Kullback-Leibler divergence (KLD), a modified log-likelihood ratio (LLR), a support vector machine, and the Bayes Factor. In one embodiment, the classifier is a learning machine selected from the group consisting of a support vector machine, an extreme learning machine, and an interactive learning machine. In one embodiment, the classifier is a pre-trained support vector machine.

The classifier may be trained with training data that are structured as described above and further structured by applying a system-defined ontology for epilepsy. The ontology for epilepsy comprises term classes which describe selected medical concepts related to the diagnosis, treatment, and prognosis of epilepsy. The ontology further captures the relationships between these concepts and contains properties of each concept describing the features or attributes of the concept. For example, the ontology captures the relationships between various forms of epilepsy and clinical observations relevant to the diagnosis of those forms, the relationships between the forms of epilepsy and typical therapeutic interventions, and the relationships between the forms of epilepsy, typical therapeutic interventions, and expected outcomes.

In one embodiment, the ontology for epilepsy comprises one or more, or all, of the term classes selected from seizure type, etiology, epilepsy syndrome by age, epilepsy classification, treatment, and diagnostic testing. Each term class is further divided into 1, 2, 3, or more subclasses, which may themselves be further divided into 1, 2, or more subclasses until the desired level of granularity is reached. For example, the term class "seizure type" may be divided into three subclasses: focal seizures, generalized seizures, and unclassified seizures. In turn, the subclass "focal seizures" may be further divided into nine subclasses: absence seizures, myoclonic seizures, tonic-clonic seizures (in any combination), clonic seizures, tonic seizures, epileptic spasms (focal or generalized), atonic, infantile spasm, or other. And the subclass "absence seizures" may be further divided into absence-typical or absence-atypical.

In one embodiment, the ontology for epilepsy comprises one or more, or all, of the following term classes and subclasses.

| Term Class | Subclass 1 | Subclass 2 |
|---|---|---|
| seizure type | Focal seizures | Without impairment of consciousness or responsiveness |
| | | With impairment of consciousness or responsiveness |
| | | Evolving to a bilateral, convulsive seizure |
| | | Other |
| | Generalized seizures | |
| | | Absence |
| | | Myoclonic |
| | | Clonic |
| | | Tonic |
| | | Epileptic Spasms |
| | Unclassified seizures | Atonic |
| | Seizure free since last visit | Infantile spasm |
| | Not seizure free since last visit | |
| | | Hourly seizures |
| | | Daily seizures |
| | | Weekly seizures |
| | | Monthly seizures |
| | | Yearly seizures |
| etiology | Structural or metabolic | Structural |
| | | Metabolic |
| | Genetic or presumed genetic | Proven genetic symptomatic etiology |
| | | Presumed genetic symptomatic etiology |
| | | Proven genetic idiopathic etiology |

(continued)

| Term Class | Subclass 1 | Subclass 2 |
|---|---|---|
| | | Presumed genetic idiopathic etiology |
| epilepsy syndrome by age | | |
| | Neonatal | Benign familial neonatal epilepsy |
| | | Ohtahara syndrome |
| | Infancy | Early myoclonic encephalopathy |
| | | Benign infantile epilepsy |
| | | West syndromes |
| | | Dravet syndrome |
| | | Myoclonic epilepsy in infancy |
| | Childhood | Epilepsy of infancy with migrating focal seizures |
| | | Febrile seizure plus |
| | Adolescence-Adult | Epilepsy with myoclonic atonic seizures |
| | | Epilepsy with myoclonic absences |
| | | Epilepsy with myoclonic absences |
| | | Juvenile absence epilepsy |
| | | Epilepsy with generalized tonic-clonic seizures alone |
| epilepsy classification | Localization related epilepsies | Temporal lobe |
| | | Parietal lobe |
| | Generalized Epilepsies | |
| treatment | Drug treatments not for rescue | Barbiturates |
| | | Benzodiazepines |
| | | Carbonic anhydrase inhibitors |
| | | Carboxamides |
| | Other types of treatments | GABA analogs |
| | | Ketogenic diet |
| | | Surgery |
| diagnostic testing | EEG | Normal |
| | | Abnormal |
| | Neuroimaging | Normal |
| | | Abnormal |

In one embodiment, the term classes or subclasses of the epilepsy ontology further comprise one or more of the following terms: other, none, unclear from text, and no other information available. In one embodiment, the term classes or subclasses comprise the ICD-9-CM codes for epilepsy classification (*see e.g.*, Table 6).

In one embodiment, the epilepsy ontology further comprises one or more episodic classes that describe concepts that capture information from a patient's prior visits including, for example, seizure free since last visit, not seizure free since last visit; classes that describe concepts relating to the past frequency of seizures including, for example, hourly, daily, weekly, monthly, and yearly; and other frequency of seizures, and classes that describe concepts relating to the patient's historical drug treatment data, including, for example, used as previous treatment, started as new treatment, dose not changed, dose decreased, dose increased, treatment discontinued, and treatment listed as option.

The training data is mapped to the system-defined ontology. The mapping can be performed, for example, by one or more human experts, or it can be performed by a computer-implemented method, such as a natural language processing method, or by a combination of human annotation and computer-implemented methods. In one embodiment, natural language processing tools are utilized for retrieving data represented by the concepts of the ontology from a database of electronic records. The electronic records may be contained, for example, in a database or data warehouse of clinical data or electronic medical records. The training data may be updated periodically to improve the performance of the SVM

The training data consists of n-grams extracted from two corpora of clinical text, a first corpora from patients who had intractable epilepsy ("the intractable group") and a second corpora from patients who had non-intractable epilepsy ("the non-intractable group"). The intractable group consists of data extracted from the clinical notes of patients with epilepsy who were referred for, and eventually underwent, epilepsy surgery. The non-intractable group consists of data extracted from the clinical notes of patients with epilepsy who were responsive to medications and never referred for surgical evaluation. In one embodiment, the clinical text is extracted from EHRs contained within an electronic medical record system using a series of scripts, such as PL/SQL scripts. Following n-gram extraction, the data is structured as described above and the structured data is used to train the classifier. Preferably the data used for training is obtained from a corpus of clinical text where each document in the corpus satisfies each of the following criteria: it was created for an office visit, it is over 100 characters in length, it comprises an ICD-9-CM code for epilepsy, and it is signed by an attending clinician, resident, fellow, or nurse practioner. In addition, each patient represented in the corpus is preferably represented by at least 4 documents, each from a separate office visit.

In one embodiment, the method further comprises a step of de-identifying the clinical text to be included in the training set. The de-identification process may include both automated methods and manual review.

Various implementations of the subject matter described herein can be realized/implemented in digital electronic circuitry, integrated circuitry, specially designed application specific integrated circuits (ASICs), computer hardware, firmware, software, and/or combinations thereof. These various implementations can be implemented in one or more computer programs. These computer programs can be executable and/or interpreted on a programmable system. The programmable system can include at least one programmable processor, which can be a special purpose or a general purpose processor. The at least one programmable processor can be coupled to a storage system, at least one input device, and at least one output device. The at least one programmable processor can receive data and instructions from, and can transmit data and instructions to, the storage system, the at least one input device, and the at least one output device.

These computer programs (also known as programs, software, software applications or code) can include machine instructions for a programmable processor, and can be implemented in a high-level procedural and/or object-oriented programming language, and/or in assembly/machine language. As can be used herein, the term "machine-readable medium" can refer to any computer program product, apparatus and/or device (for example, magnetic discs, optical disks, memory, programmable logic devices (PLDs)) used to provide machine instructions and/or data to a programmable processor, including a machine-readable medium that can receive machine instructions as a machine-readable signal. The term "machine-readable signal" can refer to any signal used to provide machine instructions and/or data to a programmable processor.

To provide for interaction with a user, the subject matter described herein can be implemented on a computer that can display data to one or more users on a display device, such as a cathode ray tube (CRT) device, a liquid crystal display (LCD) monitor, a light emitting diode (LED) monitor, or any other display device. The computer can receive data from the one or more users via a keyboard, a mouse, a trackball, a joystick, or any other input device. To provide for interaction with the user, other devices can also be provided, such as devices operating based on user feedback, which can include sensory feedback, such as visual feedback, auditory feedback, tactile feedback, and any other feedback. The input from the user can be received in any form, such as acoustic input, speech input, tactile input, or any other input.

The subject matter described herein can be implemented in a computing system that can include at least one of a back-end component, a middleware component, a front-end component, and one or more combinations thereof. The back-end component can be a data server. The middleware component can be an application server. The front-end component can be a client computer having a graphical user interface or a web browser, through which a user can interact with an implementation of the subject matter described herein. The components of the system can be interconnected by any form or medium of digital data communication, such as a communication network. Examples of communication networks can include a local area network, a wide area network, internet, intranet, Bluetooth network, infrared network, or other networks.

The computing system can include clients and servers. A client and server can be generally remote from each other and can interact through a communication network. The relationship of client and server can arise by virtue of computer programs running on the respective computers and having a client-server relationship with each other.

Example 1: Classification of clinical notes to identify epilepsy patients who are candidates for surgery

This research analyzed the clinical notes of epilepsy patients using techniques from corpus linguistics and machine learning and predicted which patients are candidates for neurosurgery, *i.e.* have intractable epilepsy, and which are not.

In this example, formation-theoretic and machine learning techniques are used to determine whether sets of clinical notes from patients with intractable and non-intractable epilepsy are different, if they are different, how they differ. The results of this work demonstrate that clinical notes from patients with intractable and non-intractable epilepsy are different and that it is possible to predict from an early stage of treatment which patients will fall into one of these two categories based only on textual data. It typically takes about 6 years for a clinician to determine that a patient should be referred for surgery. The present methods reduce this time period to about four years, which is a significant reduction. Accordingly, the methods described here are useful for clinical decision support for epilepsy patients.

Two bodies of clinical text were used for this example. The first from patients with epilepsy who were referred for, and eventually underwent, epilepsy surgery ("intractable group"). The second from patients with epilepsy who were responsive to medications and never referred for surgical evaluation ("non-intractable group"). Two methods for detecting differences in the clinical text were evaluated to determine whether the two groups of clinical text could be distinguished. The methods used were Kullback-Leibler Divergence (KLD) and a Support Vector Machine (SVM).

KLD is a traditional statistical method used to determine whether or not two sets of n-grams are derived from the same distribution. KLD is the relative entropy of two probability mass functions, *i.e.*, a measure of how different two probability distributions are over the same event space (Manning & Schuetze, 1999). This measure has been used previously to assess the similarity of corpora (Verspoor, Cohen, & Hunter, BMC Bioinfo. 10(1) 2009). Details of the calculation of KLD are given in the methods section. KLD has a lower bound of zero; with a value of zero, the two document sets would be identical. A value of 0.005 is assumed to correspond to near-identity.

For both methods, neurology clinic notes were extracted from the electronic medical record system (EPIC/Clarity) using a series of PL/SQL scripts. To be included, the notes had to have been created for an office visit, be over 100 characters in length, and have one of the ICD-9-CM codes for epilepsy classification listed in Table 6. In addition, each note had to be signed by an attending clinician, resident, fellow, or nurse practitioner, and each patient was required to have at least one visit per year between 2009 and 2012 (for a minimum of four visits). Records were sampled from the two groups at three time periods before the "zero point", the date at which patients were either referred for surgery (intractable group) or the date of last seizure (non-intractable group). Table 1 shows the distribution of patients and clinic notes. In the table, a minus sign indicates the period before surgery referral date for intractable epilepsy patients and before last seizure for non-intractable patients. A plus sign indicates the period after surgery referral for intractable epilepsy patients and after last seizure for non-intractable patients. Zero is the surgery referral date or date of last seizure for the two populations, respectively.

Table 1: Progress note and patient counts (in parentheses) for each time period.

|                | Non-Intractable | Intractable |
| -------------- | --------------- | ----------- |
| -12 to 0       | 355 (127)       | 641 (155)   |
| -6 to +6       | 453 (128)       | **898** (**155**) |
| 0 to +12 months | 454 (132)      | **882** (149) |

The notes were then de-identified using a combination of automatic output from the MITRE Identification Scrubber Tool (MIST) and manual review. After de-identification, the n-gram frequencies were extracted from each note, and all characters in the note were changed to lower case. Age, patient name, location, hospital name, any initials, patient identification numbers, phone numbers, URLs, and miscellaneous protected information such as account numbers and room numbers were replaced with 'AGE,' 'NAME,' 'LOCATION,' 'HOSPITAL,' 'INITIALS,' 'ID,' 'PHONE,' 'URL,' and 'OTHER,' respectively. Non-ASCII and non-alphanumeric characters were then removed, as were words from The National Library of Medicine stopword list, and all numbers were changed to 'NUMB.' All n-grams that occurred less than nine times within the whole data set were removed. Finally, the notes were mapped to an ontology for epilepsy developed by the inventors.

n-grams were extracted from the clinical text and structured as described above before applying either the KLD-based method or the SVM to determine whether the two document collections were different (or differentiable). Features for both the calculation of KLD and the machine learning experiment were unigrams, bigrams, trigrams, and quadrigrams.

KLD compares probability distribution of words or n-grams between different datasets DKL(P\\Q). In particular, it measures how much information is lost if distribution Q is used to approximate distribution P. This method, however, gives an asymmetric dissimilarity measure. Jensen-Shannon divergence (DJS) is probably the most popular symmetrization of DKL.

By Zipfs law any corpus of natural language will have a very long tail of infrequent words. To account for this effect, DJS were used for the top N most frequent words/n-grams. Laplace smoothing was used to account for words or n-grams that did not appear in one of the corpora.

Terms that distinguished one corpus from another were also accounted for using a metamorphic DJS test, log-likelihood ratios, and weighted SVM features.

For the classification part of the experiment, an implementation of the libsvm support vector machine package that was ported to R (Dimitriadou et al., 2011) was used. Features were extracted as described above. A cosine kernel was used. The optimal C regularization parameter was estimated on a scale from 2-1 to 215

Next, in the experiment, a variety of methods were used to characterize differences between the document sets: log-likelihood ratio, SVM normal vector components, and a technique adapted from metamorphic testing (Murphy and Kaiser, 2008).

The intuition behind metamorphic testing is that given some output for a given input, it should be possible to predict in general terms what the effect of some alternation in the input should be on the output. For example, given some KLD for some set of features, it is possible to predict how KLD will change if a feature is added to or subtracted from the feature vector. This observation was adapted by iteratively subtracting all features one by one and ranking them according to how much of an effect on the KLD their removal had.

From the experimental data, Table 2 shows the KLD, calculated as Jensen-Shannon divergence, for three overlapping time periods-the year preceding surgery referral, the period from 6 months before surgery referral to six months after surgery referral, and the year following surgery referral, for the intractable epilepsy patients; and, for the non-intractable epilepsy patients, the same time periods with reference to the last seizure date. In the table, results are shown for the period 1 year before, 6 months before and 6 months after, and one year after surgery referral for the intractable epilepsy patients and the last seizure for non-intractable patients. 0 represents the date of surgery referral for the intractable epilepsy patients and date of last seizure for the non-intractable patients. As can be seen in the left-most column (-12 to 0) in Table 2, at one year prior, the clinic notes of patients who will require surgery and patients who will not require surgery can be easily discriminated by KLD. At all feature cutoffs (i.e. counts of top n-grams), the KLD is well above the .005 level that indicates near-identity. Any null hypothesis that there is no difference between the two collections of clinic notes can be rejected. If the -6 to +6 and 0 to +12 time periods are examined, it can be seen that the KLD increases as we reach and then pass the period of surgery (or move into the year following the last seizure, for the non-intractable patients), indicating that the difference between the two collections is more pronounced as treatment progresses.

Table 2: Kullback-Leibler divergence (calculated as Jensen-Shannon divergence) for difference between progress notes of the two groups of patients.

| n-grams | -12 to 0 months | -6 to +6 months | 0 to +12 months |
|---|---|---|---|
| 125 | 0.0242 | 0.0430 | 0.0544 |
| 250 | 0.0226 | 0.0358 | 0.0440 |
| 500 | 0.0177 | 0.0264 | 0.0319 |
| 1000 | 0.0208 | 0.0287 | 0.0346 |
| 2000 | 0.0209 | 0.0271 | 0.0313 |
| 4000 | 0.0159 | 0.0198 | 0.0232 |
| 8000 | 0.0100 | 0.0123 | 0.0144 |

These data show that the two major paths in epilepsy care (intractable patients in whom surgery may be necessary and non-intractable patients in whom surgery is not necessary) can, at some point in time, be distinguished based upon clinical notes alone.

Table 3 shows the results of building support vector machines with the experimental data to classify individual notes as belonging to the intractable or the non-intractable epilepsy group. The time periods are as described above. The number of features is varied by row. For each cell, the average F-measure from 20-fold cross-validation is shown.

Table 3: Average F-1 for the three time periods described above, with increasing numbers of features.

| n-grams | -12 to 0 months | -6 to +6 months | 0 to +12 months |
|---|---|---|---|
| 125 | 0.8856 | 0.9285 | 0.9558 |
| 250 | 0.8963 | 0.9389 | 0.9603 |
| 500 | 0.9109 | 0.9553 | 0.9677 |
| 1000 | 0.9258 | 0.9607 | 0.9734 |
| 2000 | 0.9361 | 0.9659 | 0.9796 |
| 4000 | 0.9437 | 0.9703 | 0.9821 |

(continued)

| n-grams | -12 to 0 months | -6 to +6 months | 0 to +12 months |
|---------|-----------------|-----------------|-----------------|
| 8000 | 0.9504 | 0.9705 | 0.9831 |

As can be seen in the left-most column (-12 to 0), at one year prior to referral to surgery, referral date, or last seizure, the patients who will become intractable epilepsy patients can be distinguished from the patients who will become non-intractable epilepsy patients purely on the basis of natural language processing-based classification with an F-measure as high as 0.95. This is consistent with the results from KLD showing that the two document sets are indeed different, and further illustrates that this difference can be used to predict which patients will require surgical intervention.

Tables 4 and 5 show the experimental results of three classification methods for differentiating between the document collections representing the two patient populations. The methodology for each is described above. Table 4 shows features for the -12 to 0 periods with the 125 most frequent features. The JSMT and LLR statistics give values greater than zero. Sign (+/-) indicates which corpus has higher relative frequency of the feature: a positive value indicates that the relative frequency of the feature is greater in the intractable group, while a negative value indicates that the relative frequency of the feature is greater in the non-intractable group. The last row shows the correlation between two different ranking statistics. Table 5 shows features for the -12 to 0 periods with the 8,000 most frequent features. The JSMT and LLR statistics give values greater than zero. We add sign to indicate which corpus has higher relative frequency of the feature: a positive value indicates that the relative frequency of the feature is greater in the intractable group, while a negative value indicates that the relative frequency of the feature is greater in the non-intractable group. The last row shows the correlation between two different ranking statistics.

Table 4: Comparison of three different methods for finding the strongest differentiating features (125 most frequent features)

| JS metamorphic test (JSMT) | Log-likelihood ratio (LLR) | SVM normal vector components (SVMW) |
|-----------------------------|----------------------------|-------------------------------------|
| none = 0.003256 | none = 623.702323 | bilaterally = -19.695683 |
| NUMB = -0.003043 | family = -445.117177 | age.NUMB = 17.5044 |
| NUMB.NUMB.NUMB.NUMB = 0.002228 | NUMB .NUMB .NUMB .NUMB = 422.953816 | first = -16.689728 |
| NUMB.NUMB = -0.001282 | normal = -244.603033 | review = 13.848571 |
| problems = -0.000955 | problems = -207.02113 | awake = -13.410366 |
| left = 0.000839 | left = 176.434519 | based = -13.343644 |
| bid = 0.000684 | bid = 142.105691 | mother = -13.34311 |
| detailed = -0.000599 | NUMB = 136.255678 | clinic = 13.29439 |
| normal = -0.000564 | detailed = -133.012908 | hpi = 12.87825 |
| right = 0.000525 | right = 120.453596 | negative = 12.61737 |
| risks = -0.000522 | seizure = -120.047686 | brain = -11.9009 |
| including = -0.000503 | including = -119.061518 | lower= -11.80371 |
| additional = -0.000412 | risks = -116.54325 | including = -11.2368 |
| concerns = -0.00041 | concerns = -101.36611 | family.history = -10.90465 |
| clear = 0.000351 | additional = -95.880792 | effects = 10.7428 |
| history = 0.000323 | clear = 83.84817 | documented = -10.6560 |
| brain = -0.000278 | brain = -74.26722 | significant = 10.60867 |

(continued)

| JS metamorphic test (JSMT) | Log-likelihood ratio (LLR) | SVM normal vector components (SVMW) |
|---|---|---|
| seizure = -0.000268 one = 0.000253 | seizures = 71.937757 | side.effects = -10.5587 |
| | one = 65.203819 | follow = -10.45960 |
| seizure = -0.000268 | epilepsy = 46.383564 | neurology = -10.17 |
| Spearman correlation between | Spearman correlation between | Spearman correlation between |
| JSMT and LLR = 0.1717 | LLR and SVMW = 0.2259 | SVMW and JSMT = -0.0708 |

Table 5: Comparison of three different methods for finding the strongest differentiating features (8,000 most frequent features)

| JS metamorphic test (JSMT) | Log-likelihood ratio (LLR) | SVM normal vector components (SVMW) |
|---|---|---|
| family = -2e-04 | family = -830.329965 | john = -10.913326 |
| normal = -0.000171 | normal = -745.882086 | pep = -10.214928 |
| problems = -9.7e-05 | problems = -386.238711 | carnitine = -9.973413 |
| seizure = -8.9e-05 | seizure = -369.342334 | lamotrigine = 9.95866 |
| none = 8.9e-05 | none = 337.461504 | increase = 9.600876 |
| detailed = -6.9e-05 | detailed = -262.240496 | jane = -9.59724 |
| NUMB.NUMB.NUMB.NUMB = 6.6e-05 | including = -255.076808 | johnson = 8.686167 |
| including = -6.6e-05 | additional.concerns.noted = -246.603655 | office = -8.304699 |
| *additional.concerns.noted = -6.5e-05* | *concerns.noted = -246.603655* | *po = -8.142393* |
| concerns.noted = -6.5e-05 | additional.concerns = 243.353912 | precautions = 8.101786 |
| additional.concerns = -6.4e-05 | NUMB.NUMB.NUMB.NUMB = 238.0657 | excellentcontrol = -7.86907 |
| risks = -6.2e-05 | risks = -232.741511 | twice = -7.817349 |
| concerns = -6e-05 | concerns = -228.805299 | excellent = -7.575003 |
| additional = -5.5e-05 | additional = -204.462411 | NUMB.seizure = -7.421679 |
| brain = -4.9e-05 | brain = -182.41334 | discussed = -7.379607 |
| surgery = 4.6e-05 | NUMB = -162.992065 | pat= -7.315927 |
| minutes = -3.9e-05 | surgery = 153.64606 | re = -7.247682 |
| NUMB.minutes = -3.8e-05 | minutes = -142.7619 | continue = -7.228999 |
| cliff = -3.8e-05 | NUMB.minutes = -134.048116 | cbc = -7.137903 |
| idiopathic = -3.3e-05 | diff = -131.3882 | smith = 7.131959 |
| Spearman correlation between JSMT and LLR = 0.9056 | Spearman correlation between LLR and SVMW = 0.07187 | Spearman correlation between SVMW and JSMT = 0.04894 |

Impressionistically, two trends emerge. One is that more clearly clinically significant features are shown to have strong discriminatory power when the 8,000 most frequent features are used than when the 125 most frequent features are used. The other trend is that the SVM classifier does a better job of picking out clinically relevant features.

KLD varies with the number of words considered. When the vocabularies of two document sets (a first multitude of clinical notes pertaining to a group patients known to have intractable epilepsy and a second multitude of clinical notes pertaining to a group of patients known to have non-intractable epilepsy) are merged and the words are ordered by overall frequency, the further down the list we go, the higher the KLD can be expected to be. This is because the highest-frequency words in the combined set will generally be frequent in both source corpora, and therefore carry similar probability mass. As we progress further down the list of frequency-ranked words, we include progressively less-common words, with diverse usage patterns, which are likely to reflect the differences between the two document sets, if there are any. Thus, the KLD will rise.

To understand the intuition here, one may look back at the KLD when just the 50 most-common words are considered. These will likely be primarily function words, and their distributions are unlikely to differ much between the two document sets unless the syntax of the two corpora is radically different. Beyond this set of very frequent common words will be words that may be relatively frequent in one set as compared to the other, contributing to divergence between the sets.

In Table 2, the observed behavior for the two document collections used in the experiment does not follow this expected pattern. It was observed that while the null hypothesis of similarity of the two document sets can clearly be rejected on the basis of these results, the divergence overall is substantially lower when more words are considered (>2000 top n-grams) than the results observed by (Verspoor et al., BMC Bioinfo. 10(1) 2009) for two corpora determined in that work to be highly similar.

This behavior may be attributed to two factors. The first is that both document sets derive from a single department within a single hospital; a relatively small number of doctors are responsible for authoring the notes and there may exist specific hospital protocols related to their content. The second is that the clinical contexts from which the two document sets are derived are highly related, in that all the patients are epilepsy patients. While it has been demonstrated that there are clear differences between the two sets, it is also to be expected that they would have many words in common. The nature of clinical notes combined with the shared disease context results in generally consistent vocabulary and hence low overall divergence.

Table 3 demonstrates that classifier performance increases as the number of features increases. This indicates that as more terms are considered, the basis for differentiating between the two different document collections is stronger.

Examining the SVM normal vector components (SVMW) in Tables 4 and 5, it can be seen that both unigrams and bigrams are useful in differentiation between the two patient populations. While no trigrams or quadrigrams appear in this table, they may in fact contribute to classifier performance.

This first set of experiments using KLD and classification by machine learning support rejection of the null hypothesis of no detectable differences between the clinic notes of patients who will progress to the diagnosis of intractable epilepsy and patients who do not progress to the diagnosis of intractable epilepsy. The results show that a prediction can be made from an early stage of treatment which patients will fall into these two classes based only on textual data from the neurology clinic notes. SVM classification confirms the results of the information-theoretic measures, uses less data, and may need just a single run.

Example 2: SVM can classify clinical notes from different hospitals

As proof of concept that an SVM could be used clinically to identify epilepsy patients who are candidates for surgery, we trained an SVM using epilepsy progress notes from different hospitals. The SVM classifies the notes based on the frequencies of (strings of) words (n-grams) in the notes. The common vocabulary is therefore strictly defined by those n-grams that are associated with the classifications. The SVM is trained to classify each progress note as belonging to a patient with one of three broadly defined categories of epilepsy: PE, GE, and UE. Due to the lack of consensus in their annotation, the epilepsy progress notes are defined by the ICD-9-CM codes assigned to them by their authors with GE defined by 345.00, 345.01, 345.10, 345.11, and 345.2; PE defined by 345.40, 345.41, 345.50, 345.51, 345.70, and 345.71; and UE defined by 345.80, 345.81, 345.90, and 345.91. Note that the codes themselves never occur in the notes, and since the clinicians are not required to use any controlled vocabulary, the text strings associated with the codes most likely never occur in the notes either.

Table 6 summarizes the ICD-9-CM codes and lists the numbers of progress notes available for classification for each hospital. As there are sizable variations in the number of notes between the three epilepsy types, using them all would result in sample-size effects that could be confused with inter-hospital differences in vocabulary. We therefore fix the training and data sample sizes to 90 documents per hospital per epilepsy classification in the training set, and to 45 documents per hospital per epilepsy classification in the testing data set. The training set is used for two purposes: for cross-validation of the parameter space and for building the optimal classifier. The test set (i.e., 'remaining hospital(s)') is withheld until the optimal classifier is built on the full training data.

**Table 6:** The ICD-9-CM codes associated with each type of epilepsy diagnosis, and the corresponding number of clinical notes from each hospital

| Epilepsy classification | ICD-9-CM codes | CCHMC | CHCO | CHOP |
|---|---|---|---|---|
| Partial epilepsy | 345.40, 345.41, 345.50, 345.51, 345.70, 345.71 | 303 | 128 | 269 |
| Generalized epilepsy | 345.00,345.01,345.10, 345.11, 345.2 | 99 | 163 | 129 |
| Unclassified epilepsy | 345.80, 345.81, 345.90, 345.91 | 200 | 117 | 121 |
| Data missing | 345.3, 345.60, 345.61 | 12 | 25 | 32 |

CCHMC, Cincinnati Children's Hospital Medical Center; CHCO, Children's Hospital Colorado; CHOP, Children's Hospital of Philadelphia.

To validate the gold standard in the face of known problems with practitioner-assigned ICD-9-CM codes, a random sample of 24 notes from each category was assembled. Each note was annotated by two physicians, with each physician only coding the notes from the hospital(s) other than their own. This process resulted in a Krippendorff's $\alpha$ of 0.691 (with chance agreement of 1/4), suggesting that the gold standard is of good quality. When we combined the post hoc coding with the coding done by the authors of the notes, Krippendorff's $\alpha$ slightly decreased to 0.626. The documents are represented by their unigrams, bigrams, and trigrams, which serve as features for the SVM. We found that the inclusion of n-grams with n larger than 3 decreases classification accuracy (the F1 score described below) during training, probably due to over-fitting. The extraction of n-grams is described in the following section. This is the most basic representation that could be used. An alternative approach would be to use semantic features, rather than surface linguistic features, by running a term extraction engine such as MetaMap, cTAKES, or ConceptMapper, and then classifying based on the extracted semantic concepts. As will be seen, good classification can be obtained with the simpler approach. Furthermore, abstraction of semantic concepts has the effect of making the three hospitals more homogeneous, so the surface linguistic features provide a more stringent evaluation of the hypothesis.

N-gram extraction

We used the electronic health records from the neurology departments of three different hospitals: the Cincinnati Children's Hospital Medical Center (CCHMC), Children's Hospital Colorado (CHCO), and Children's Hospital of Philadelphia (CHOP). The progress notes were required to have been created for an office visit, be over 100 characters in length, and have one of the ICD-9-CM codes listed in table 1. Further, each note had to be signed by an attending clinician, resident, fellow, or nurse practitioner. Lastly, each patient was required to have at least one visit per year between 2009 and 2012 (for a minimum of four visits). Overall, 551, 614, and 433 progress notes from CHOP, CCHMC, and CHCO, respectively, satisfied all of the selection criteria. The notes were then de-identified and structured as described in Example 1.

Classification

The SVMs were trained using 90 documents for each of the three epilepsy types, with as many as 23,017 n-grams, and optimized using an F1 score defined by

$$F_1 = \frac{2t_n^2}{(t_n + f_p)(t_n + f_n)}$$

where $t_n$ is the number of true positives, $f_p$ is the number of false positives, and $f_n$ is the number of false negatives.

N-grams were weighted based on one of two weighting schemes. The schemes were selected using cross-validation methods, among other parameters. Ultimately, the SVM was optimized over the cost regularization parameter (the C parameter), the number of top-ranked n-grams to use for the SVM input (N), and the ranking method and n-gram weighting schemes using the 20-fold cross-validated F1 score. The cost parameter was optimized over 18 values ranging from 2-8 to 24, incremented by factors of 2. Parameter N is optimized over 25 to 213 n-grams, incremented by factors of 20.5.

The n-grams were ranked based on either information gain, information gain ratio, or the Pearson correlation coefficient. Overall, the SVM was optimized over 13 values of the C parameter, 16 values of N, 2 feature weightings, 3 feature rankings, and 20 folds. This translates to an optimization over 1,248 points in the parameter space and 24,960 runs of the SVM.

As discussed previously, the UE classification can be ambiguous. We therefore classified GE and PE for three hospitals using training samples from either one or two of the other hospitals. This gives six possible combinations of hospitals. The baseline classifier for these experiments was random class assignment, which yields F1=50%.

We also performed a second analysis assuming three possible types of epilepsy-PE, GE, and UE. Because SVMs are built for binary classification, three SVMs were trained to classify PE versus not-PE, GE versus not-GE, and UE versus not-UE, with the results being subsequently combined to effectively provide a tertiary classification. The baseline classifier for these experiments was F1=33%.

Results

Table 7 summarizes the performance of our SVM trained assuming patients are either PE or GE. It shows 20-fold cross-validated F1's and corresponding SDs for both GE and PE progress notes. The corresponding average F1's and their SDs from progress notes sampled from the hospitals not in the training set (i.e., 'remaining hospitals') are also listed along with the p value significance, which assume a random baseline classification of F1=50%. The p values show the SVM is capable of classifying PE and GE above baseline, although the p value in the case where the training sample is CCHMC and the F1 is evaluated on CHOP and CHCO is significantly smaller than in the case when the SVM is trained and evaluated with other training and testing data sets. Note that the F1's are all above approximately 75% when the SVM is trained on two hospitals. Also, training with two hospitals yields an increase of about 10.4% in F1. The other effect of adding a second hospital is the decreased gap between training F1 and testing F1. The gap 0.871-0.725=0.146 decreases to 0.899-0.829=0.070, yielding a 7.6% improvement. The last column shows the p value significance of the result compared to the largest class baseline F1=0.5. Systematic improvement when two hospitals are used is highlighted in bold, and the sample size is the same when one and two hospitals are used. All three effects suggest that two hospitals are enough to make the third one more similar.

**Table 7:** Results from the classification of partial epilepsy and generalized epilepsy in epilepsy progress notes

| Hospital used for training | Average F1 (training) | F1 SD (training) | Average F1 (remaining hospitals) | F1 SD (remaining hospitals) | p Value from baseline (remaining hospitals) |
|---|---|---|---|---|---|
| CCHMC | 0.865 | 0.213 | 0.691 | 0.095 | 0.043 |
| CHOP | 0.926 | 0.149 | 0.729 | 0.014 | <0.001 |
| CHCO | 0.823 | 0.224 | 0.754 | 0.062 | <0.001 |
| One-hospital average | 0.871 | 0.195 | 0.725 | 0.070 | 0.001 |
| CCHMC and CHOP | 0.913 | 0.100 | 0.817 | 0.047 | <0.001 |
| CCHMC and CHCO | 0.904 | 0.097 | 0.807 | 0.031 | <0.001 |
| CHOP and CHCO | 0.904 | 0.097 | 0.807 | 0.031 | <0.001 |
| Two-hospital average | 0.899 | 0.105 | 0.829 | 0.047 | <0.001 |

CCHMC, Cincinnati Children's Hospital Medical Center; CHCO, Children's Hospital Colorado;
CHOP, Children's Hospital of Philadelphia.

The results from our second study, where we include patients with UE, are shown in Table 8. The first column lists the hospital(s) used to optimize the support vector machine. The second and third columns list the 20-fold cross-validated average F1 and corresponding SDs of the training samples, respectively. The fourth and fifth columns list the average F1 and corresponding SDs for the remaining hospital(s). The last column shows the p value significance of the result compared to the largest class baseline F1 $\approx$ 0.333. Systematic improvement when two hospitals are used is highlighted in bold, and the sample size is the same when one and two hospitals are used. The F1 scores are all above the baseline value of 33%, although somewhat marginally. As before, there is a 10.4% improvement in F1 when a second hospital is added to the training set and the F1 gap between the training and testing sets decreases from 0.289 to 0.216, which is an improvement of about 7.3%.

**Table 8:** Results from the classification of PE, GE, and UE in epilepsy progress notes

| Hospital used for training | Average F1 (training) | F1 SD (training) | Average F1 (remaining hospitals) | F1 SD (remaining hospitals) | p Value from baseline (remaining hospitals) |
|---|---|---|---|---|---|
| CCHMC | 0.647 | 0.311 | 0.417 | 0.147 | 0.567 |
| CHOP | 0.759 | 0.261 | 0.372 | 0.142 | 0.788 |
| CHCO | 0.625 | 0.327 | 0.376 | 0.143 | 0.763 |
| One hospital | 0.677 | 0.300 | 0.388 | 0.145 | 0.704 |
| CCHMC and CHOP | 0.670 | 0.169 | 0.478 | 0.097 | 0.136 |
| CCHMC and CHCO | 0.724 | 0.172 | 0.424 | 0.113 | 0.421 |
| Two hospitals | 0.708 | 0.175 | 0.492 | 0.153 | 0.298 |

CCHMC, Cincinnati Children's Hospital Medical Center; CHCO, Children's Hospital Colorado; CHOP, Children's Hospital of Philadelphia; GE, generalized epilepsy; PE, partial epilepsy; UE, unclassified epilepsy.

Although the changes in the second study are marginal, they do not contradict our previous conclusions. Most likely the notes from UE patients obscure the classification of GE and PE, as words associated with both would also appear in the UE notes.

These results show that an SVM classifier with surface linguistic features can be built that supports the rejection of our null hypothesis (which is that such an algorithm cannot be trained using epilepsy-specific notes from one hospital and then successfully used to classify epilepsy patients from another hospital) with statistical significance. We have therefore established a certain uniformity among epilepsy progress notes from three different institutions: the CCHMC, CHCO, and CHOP. The document/n-gram matrix was built using unigrams, bigrams, and trigrams, and employed for training SVM text classifiers.

These results also demonstrate that for a given (fixed) number of progress notes, the classification of patient notes from a third hospital is improved by using notes from two hospitals in the SVM training set. That is, given the choice of increasing the sample size by increasing the number of notes from a single hospital, or broadening the note pool by including notes from another hospital, our results suggest the latter is the better choice for classification. In other words, these results suggest the inclusion of a second hospital may yield an improvement. The case where the training sample is CCHMC progress notes and the model is evaluated on CHOP and CHCO progress notes gives a significance of ~5%, whereas those cases where two hospitals are included in the training set all yield an improvement over baseline that is statistically significant at a p value of <0.01.

In summary, this work establishes that there is a certain degree of uniformity of epilepsy vocabulary across different hospitals, and has developed an NLP-based machine learning technique to classify and extract information from epilepsy progress notes. This suggests that a limited number of annotated epilepsy progress notes from each hospital might be enough for developing automated extraction of epilepsy quality measures from clinical narratives.

Example 3: Comparison of Corpus Linguistics and Machine Learning Techniques in Determining Differences in Clinical Notes

<u>Summary</u>: In this study we evaluate various linguistic and machine learning methods for determining differences between clinical notes of epilepsy patients that are candidates for neurosurgery (intractable) and those who are not (non-intractable). This paper stands as a precursor for developing patient-level classification where the training set is limited and linguistic sub-domains are difficult to determine. Data are from 3,664 clinical epilepsy clinical notes. Four methods are compared: support vector machines, log-likelihood ratio, KLD, and Bayes factor. As with many natural language processing studies, a priori knowledge is absent and the data act as a proxy. The relative performance of these methods can then be evaluated based on their ability to and differences between the intractable and non-intractable patient data. These same techniques are modified to determine if n-grams that characterize the corpora's differences give insight into the performance of the methods. The results indicate that using limited number of unigrams and limited number of clinical notes, the support vector machines are optimal. Kullback-Leibler, Bayes factor and log-likelihood ratio are highly correlated methods, while support vector machines are not. All methods were able to discern sets of documents from intractable and non-intractable patients. All methods were able to find interesting clinical differences between the document sets.

The general design of the experiments is as follows. Sets of documents from intractable and non-intractable patients are divided into 5 time periods relative to the date of the last seizure and surgery referral, respectively. For each time period, four sets of corpora are generated by randomly selecting two independent sets of documents from intractable patients, and two independent sets from non-intractable patients. The four methods are then evaluated on the intractable/intractable, non-intractable/non-intractable and two independent intractable/non-intractable pairs. The procedure is then repeated many times in order to generate distributions of the KLD, LLR, SVM and BF for the intractable/intractable, non-intractable/non-intractable and intractable/non-intractable corpora pairs. We then find the overlap of the distributions of like corpora (i.e., intractable/intractable or non-intractable/non-intractable) and of di

erent corpora (intractable/non-intractable); more powerful techniques will display less overlap and, hence, better discrimination. The overlap is then evaluated for each time period, with the expectation that the discrimination should improve with time.

The four methods use unigram (word) frequencies. In the first experiments, all of the unigrams from the corpora will be utilized. It will, however, be found that using the full set of unigrams, all methods are able to discriminate between intractable and non-intractable corpora with 100% accuracy. We will then evaluate the sensitivity of the methods to the amount of data available by considering only the top 400 most frequent unigrams and limiting the number of documents in the corpora, in order to test their robustness in the face of reduced data.

In addition, to give insights into how the methods work, each method is extended to perform feature extraction in order to find those unigrams that best characterize the differences between the corpora. These features not only ensure that the methods behave "rationally" at some level, but also highlight the differences between methods.

The data set is the same as that used in Example 1. The two groups were also sampled from five time periods with six month overlaps across 3.5 years around the "zero point," the date at which patients were referred to surgery or the date of last seizure. Table 9 shows the number of patients and clinic notes for the 5 time periods considered in this paper. The "zero point" not only defines the data alignment, but also indicates a "signicant" increased divergence in language. Patients with a date of last seizure will have no changes in treatment for the first 12-24 months until weaned off medication completely. Meanwhile, the patients with the date of referral will have additional text describing the need for a battery of diagnostic tests that may qualify them as potential surgery candidates.

**Table 9:** Progress notes (in parentheses), patient counts and the number of n-grams in each time period.

| Index | Period | Intractable Pts (Notes) | Non-intractable Pts (Notes) | Max unigrams |
|---|---|---|---|---|
| 1 | +0 - +12 | 150(1157) | 124 (463) | 4933 |
| 2 | -6 - +6 | 155(1055) | 121 (441) | 4923 |
| 3 | -12 - +0 | 154(638) | 121 (338) | 4828 |
| 4 | -18 --6 | 103 (285) | 61 (147) | 4381 |
| 5 | -24--12 | 67 (185) | 39(94) | 3957 |

Feature Extraction. The features used to evaluate the differences in corpora were limited to unigrams. Otherwise, feature extraction was performed as in Example 1. Briefly, once the words were extracted from the documents, they were lower-cased, substituted with the string NUMB in the event the unigram was a numeral, and removed if a unigram was a non-ASCII character or a word found in the National Library of Medicine stopwords list.

Table 9 lists the number of unigrams found within each time period. Initially, the four methods will be evaluated using the maximum number of unigrams, with each corpus in the comparison containing 58 documents randomly selected from the document set for the given time period. However, it will be found that all four methods are equally capable of discriminating sets of intractable and non-intractable documents nearly perfectly. We then evaluate the robustness of the methods by limiting the number of unigrams to the 400 most frequently occurring unigrams and limiting the data to 34 documents per corpus. (400 is the minimum number of unigrams that can be considered and still have them all occur in at least one of the pairs of corpora.) The number of unigrams were chosen to maximize the number of unigrams while ensuring that all the unigrams appear in the corpora pairs, where each corpus contains 34 documents from either the intractable or non-intractable documents within a given time period. A significant number of unigrams are lost when more than 400 unigrams are considered.

Corpora Comparisons. With the features established, the ability of each of four methods to distinguish corpora through their word frequencies was evaluated. As discussed above, four methods were used: (1) information-theoretic approach - KLD with Jensen-Shannon divergence symmetrization and Laplace smoothing to account for words or unigrams that did not appear in one of the corora (as in Example 1 above); (2) statistical approach - a modified version of the log-likelihood ratio (LLR) commonly used for feature extraction; (3) machine learning approach - the libsvm support vector machine package ported to the R (Dimitriadou, Hornik, Leisch, Meyer, & Weingessel, 2011) statistical software environment, with a linear kernel SVM with 10-fold cross-validation to find the optimal F1 score and a C regularization parameter estimated on a scale from $2^{-11}$ to $2^{-2}$; and (4) Bayesian approach - the Bayes Factor (BF), defined as the ratio of the probability of obtaining the frequencies of n-grams from two corpora, X and Y , given that they are derived from two unique parent distributions to the probability that the pair of frequencies are derived from a single parent. Mathematically, we would expect the results from the KLD and LLR and BF to be correlated. The BF is simply an extension of the LLR, and the KLD is an be argued to be related to Bayesian approach. For instance, (Caticha & Giffin, AIP Conf. Proc., 872:31 2006) showed that the Maximum Entropy methods can be used to derive Bayes' Theorem, the cornerstone of the BF.

Characterizing differences between the document sets. Given that differences between corpora have been established, we would then want to know which n-grams are most responsible for their differences. We focus here on unigrams. The details of how the most influential unigrams are determined is dependent on the method, but the tests used to determine them fall into two general categories: metamorphic tests and single feature tests. Metamorphic tests find those n-grams that best characterize the differences in the distributions by measuring the effect on the method's discrimination when it is removed. Single-feature testing generally measures the discrimination power if a single word were used. Single feature testing simply involves narrowing each of the four methods to a single feature to determine which features best characterize the differences between corpora. Metamorphic testing. Mathematically determining the contribution of each unigram for a given method is an obvious way of finding those n-grams that most characterize differences between corpora. However, if there is a high degree of correlation between two features, it may not matter if one or both are used. Metamorphic testing, inspired by the work of (Murphy & Kaiser, 2008), is a way of finding the contribution of a feature while folding in the degree of correlation that it has with other features. In the metamorphic test, the smaller the correlation with other features, the larger the effect on the discriminant when it is removed, the larger its contribution to characterizing differences.

Results: The discriminative power of a method within a given time period was quantified as follows. Four independent corpora, each consisting of 58 documents, were randomly selected from the set of intractable (non-intractable) patient documents. One corpus was from intractable patients, labeled corpus 1 and 2, and the second corpus from non-intractable patients, labeled corpus 3 and 4. The two other corpora consist of corpus 1 and 3 and corpus 2 and 4. The discriminant for the method was then evaluated on each pair. This was repeated 20,000 times, producing distributions for intractable corpora, for non-intractable corpora, and for intractable/non-intractable (mixed) corpora.

We then calculated the number of times that the values within the mixed distributions were less than those of either the intractable or non-intractable distributions, hereafter simply referred to as the overlap. The greater this number, the greater the overlap between the distributions. Therefore, this number is hereafter referred to as the overlap. Document sampling, discrimination and overlap are all derived from hyper-dimensional feature space. To visualize step-by-step procedures we used a two dimensional Gaussian mixture data set for sampling, Euclidean distance as the discriminant and overlap as a function of the Gaussian mixture sigma parameter. All methods were able to discriminate between intractable and non-intractable corpora with 100% accuracy based on 20,000 repetitions. To then discern which method is the most robust, we considered only the most frequent unigrams and 34 documents in each corpus. The expectation was that the discrimination should increase with time. Only the SVM behaved as expected. That is, as we move back in time, documents from intractable and non-intractable group become more similar, so more overlaps between those groups are detected. However, it was found that increasing the number of unigrams and/or documents within the corpora increases the discrimination power of all the methods. The BF behaved as it should, rendering a value less than unity for corpora that are the same and larger than unity for corpora that are different. This indicates that the statistical model used in the BF, also used in the LLR and KLD, is accurate.

Tables 10 and 11 show the highest ranked features from time period 1 from the metamorphic and single feature testing using and the maximum number of unigrams listed in Table 1, respectively. Tables 12 and 13 show similar tables for time period 5. Note that the differences between those tables generated with the top most frequent unigrams and those generated with all the unigrams are different. This indicates the methods are not merely utilizing the most frequent unigrams but rather, the differences are characterized non-trivially. Further, two clinicians highlighted words in these tables that describe seizure, epilepsy and etiology. Note that all the methods use these words to varying degrees. The single KLD, meta KLD and SVW tests extract the most and about the same number of clinical words (highlighted words in Tables 2-5).

Further, Tables 10-13 show the LLR and BF single feature tests give highly correlated results, as might be expected as the BF is a mathematical extension of the LLR. Note the LLR single feature tests (Collins, Liu, & Leordeanu, IEEE Transactions 27(10):1631-1643 2005) and SVW (Guyon,Weston, Barnhill, & Vapnik, Machine Learning 46(1-3): 389-422 2002), while giving disparate results, are well understood. While the similarities between the LLR and BF are expected since they are mathematically similar, the dis-similar findings using other techniques are unexplained.

Table 14 shows the Spearman correlation coefficients between methods using the 400 most frequent unigrams. Each Spearman correlation coefficient was calculated by generating random samples from both intractable and non-intractable patients and then calculating the four discriminants for each sample. The BF and LLR show relatively high degrees of correlation. High correlation is also seen among the KLD, BF and LLR, as might be expected mathematically. The SVM is the least correlated with any of the other methods.

Table 10: Words that were found to most characterize differences between corpora using 400 unigrams and 1,620 documents per corpus with intractable versus non-intractable corpora with highlighted clinical words for time period 1.

| KLD single | LLR single | BF single | SVM single | KLD meta | LLR meta | BF meta | SVM meta | SVW single |
|---|---|---|---|---|---|---|---|---|
| NUMB | **surgery** | **surgery** | probability | NUMB | **surgery** | **surgery** | **surgery** | **surgery** |
| **concerns** | **concerns** | concerns | formal | **concerns** | **concerns** | none | **brain** | **surgical** |
| **normal** | none | none | **recurrence** | **normal** | none | **concerns** | **idiopathic** | **intractable** |
| **additional** | **additional** | **additional** | risks | **additional** | **additional** | **additional** | team | **idiopathic** |
| family | **detailed** | **detailed** | **idiosyncratic** | family | **detailed** | NUMB | **surgical** | **first** |
| **seizure** | **idiopathic** | **idiopathic** | toxicities | seizure | **idiopathic** | **detailed** | year | discussed |
| **noted** | diff | diff | antiepleptic | **noted** | diff | left | ordered | denies |
| **surgery** | risks | risks | **detailed** | **surgery** | risks | **idiopathic** | **neurology** | **neurology** |
| none | problems | problems | **dependent** | none | problems | right | due | decreased |
| problems | left | left | **aid** | problems | **left** | **following** | **few** | mother |
| **including** | **including** | **including** | **subsequent** | **including** | **including** | diff | plan | **frontal** |
| **detailed** | **normal** | **normal** | **decided** | **detailed** | **normal** | risks | **increase** | john |
| side | family | family | questions | **side** | family | **post** | speech | **brain** |
| **effects** | **noted** | **noted** | john | **effects** | **noted** | medically | social | **post** |
| **reviewed** | **following** | **following** | **detail** | **reviewed** | **following** | **revealed** | presents | female |

Results from metamorphic and single-features testing are denoted 'meta' and 'single', respectively; "cranio." means craniotomy, "ad-min." means administrative and "cardio." means cardiovascular.

Table 11: Words that were found to most characterize differences between corpora using all 4,933 unigrams and 1,620 documents/ corpus with intractable versus non-intractable corpora with highlighted clinical words for time period 1.

| KLD single | LLR single | BF single | SVM single | KLD meta | LLR meta | BF meta | SVM meta | SVW single |
|---|---|---|---|---|---|---|---|---|
| NUMB | **surgery** | **surgery** | probability | NUMB | **surgery** | **surgery** | **first** | **surgery** |
| **concerns** | **concerns** | **concerns** | formal | **concerns** | **concerns** | **concerns** | year | john |
| **normal** | none | none | **recurrence** | **normal** | none | none | school | acid |
| **additional** | **additional** | **additional** | risks | **additional** | **additional** | **additional** | temporal | **ineffective** |
| family | **detailed** | **detailed** | **idiosyncratic** | family | **detailed** | **detailed** | years | **levetiracetam** |
| **seizure** | **idiopathic** | **idiopathic** | toxicities | seizure | **idiopathic** | **idiopathic** | **eye** | **denies** |
| **noted** | **vns** | **vns** | antiepleptic | **noted** | **vns** | vns | john | discussed |
| **surgery** | diff | diff | **detailed** | **surgery** | diff | diff | plan | valproic |
| none | risks | risks | **dependent** | none | risks | risks | **reviewed** | **first** |
| problems | problems | problems | **aid** | problems | problems | problems | **age** | tube |
| **including** | **left** | **left** | **subsequent** | **including** | **left** | **including** | well | **mri** |
| detailed | including | including | **decided** | detailed | including | **left** | weight | **pain** |
| **side** | **normal** | **normal** | questions | **side** | **normal** | **cranio**. | gait | **post** |
| **effects** | family | family | john | **effects** | family | np | **movements** | **surgical** |
| **reviewed** | **cranio.** | **cranio.** | **detail** | **reviewed** | **cranio.** | panel | months | **small** |

EP 3 028 190 B1

Results from metamorphic and single-features testing are denoted 'meta' and 'single', respectively; "cranio." means craniotomy, "ad-min." means administrative and "cardio." means cardiovascular

Table 12: Words that were found to most characterize differences between corpora using 400 unigrams and 279 documents/corpus with intractable versus non-intractable with highlighted clinical words corpora for time period 5.

| KLD single | LLR single | BF single | SVM single | KLD meta | LLR meta | BF meta | SVM meta | SVW single |
|---|---|---|---|---|---|---|---|---|
| **normal** | **concerns** | **concerns** | formal | **normal** | **concerns** | numb | night | **shaking** |
| family | problems | problems | **admin.** | family | problems | none | **one** | **report** |
| **concerns** | none | none | questions | **concerns** | none | **partial** | notes | **bilaterally** |
| problems | NUMB | numb | nursing | problems | family | examin. | **increase** | bid |
| **seizure** | family | family | risks | seizure | **partial** | **concerns** | percentile | **concerns** |
| NUMB | **partial** | **partial** | explained | NUMB | NUMB | problems | confirmed | dr |
| **including** | examin. | **normal** | **detail** | **including** | examin. | **fever** | **control** | **eye** |
| **age** | **fever** | examin. | **understand** | **age** | **fever** | **revealed** | **bilaterally** | mos |
| **detailed** | **normal** | **fever** | **answered** | **detailed** | **normal** | cardio. | **concerns** | **reported** |
| **present** | treatments | treatments | probability | **present** | treatments | treatments | seen | **change** |
| **brain** | **admin.** | **admin.** | documented | **brain** | **admin.** | family | days | **back** |
| risks | nursing | nursing | **dependent** | risks | nursing | **admin.** | medications | father |
| **upper** | **present** | **present** | **idiosyncratic** | **upper** | **present** | nursing | presents | **control** |
| **fever** | **revealed** | **revealed** | toxicities | **fever** | **revealed** | months | current | **brain** |
| history | cardio. | risks | ix | history | cardio. | psychiatric | **time** | problems |

EP 3 028 190 B1

Results from metamorphic and single-features testing are denoted 'meta' and 'single', respectively; "cranio." means craniotomy, "ad-min." means administrative and "cardio." means cardiovascular.

Table 13: Words that were found to most characterize differences between corpora using all 3,957 unigrams and 279 documents/corpus with intractable versus non-intractable corpora with highlighted clinical words for time period 5.

| KLD single | LLR single | BF single | SVM single | KLD meta | LLR meta | BF meta | SVM meta | SVW single |
|---|---|---|---|---|---|---|---|---|
| **normal** | **lamictal** | **lamictal** | formal | **normal** | **lamictal** | **lamictal** | **left** | **report** |
| family | **concerns** | **concerns** | **admin.** | family | **concerns** | **topamax** | school | **call** |
| **concerns** | **topamax** | **topamax** | questions | **concerns** | **topamax** | concerns | **back** | **result** |
| problems | problems | problems | nursing | problems | problems | problems | absence | platelets |
| **seizure** | none | none | risks | **seizure** | none | **assistant** | md | bid |
| NUMB | NUMB | NUMB | explained | NUMB | family | **partial** | function | **begin** |
| **including** | family | family | **detail** | **including** | **assistant** | examin. | **change** | **shaking** |
| **age** | **assistant** | **assistant** | **understand** | **age** | **partial** | **fever** | months | **seizures** |
| **detailed** | **partial** | **partial** | **answered** | **detailed** | NUMB | **final** | **seizure** | **back** |
| **present** | examin. | **normal** | probability | **present** | examin. | **depakote** | **extremities** | john |
| **brain** | **fever** | examin. | documented | **brain** | **fever** | none | facial | **concerns** |
| risks | **normal** | **fever** | **dependent** | risks | **final** | treatments | gait | problems |
| **upper** | **final** | **final** | **idiosyncratic** | **upper** | **normal** | np | **tone** | **consistent** |
| **fever** | **depakote** | **depakote** | toxicities | **fever** | **depakote** | trileptal | current | plan |
| history | treatments | treatments | ix | history | treatments | **admin.** | discussed | cincinnati |

EP 3 028 190 B1

Results from metamorphic and single-features testing are denoted 'meta' and 'single', respectively; "cranio." means craniotomy, "ad-min." means administrative and "cardio." means cardiovascular.

Table 14. Spearman correlation coefficient between sampled discriminants for all periods of time when using all unigrams and 2000 repetitions.

|  | BF | KLD | LLR | SVM |
|---|---|---|---|---|
| BF | 1.0000 | 0.9487 | 0.9597 | 0.8561 |
| KLD | 0.9487 | 1.0000 | 0.9447 | 0.8746 |
| LLR | 0.9597 | 0.9447 | 1.0000 | 0.8604 |
| SVM | 0.8561 | 0.8746 | 0.8604 | 1.0000 |

Conclusions. All methods were able to discern sets of documents from intractable and non-intractable patients with 100% accuracy (based on 20,000 repetitions) when a relatively large number of documents (i.e. 58) and all of the unigrams were used. When testing the robustness of the methods by limiting the number of documents and unigrams and thereby limiting the data available to the methods, it was found that only the SVM maintained its high performance. These findings support our other evidence that SVM does not require large samples. In fact, the data representing the margin between the two corpora are sufficient and the rest can be discarded. Increasing the number of documents and/or number of unigrams increases the ability of all of the methods to discriminate between corpora. While the SVM performs better than the other methods, it is unable to quantify similarity between corpora in the event that differences are not found. Even though SVM single, SVM meta and SVW are derived from the same discriminative method, they discover very different unigrams. SVW shows some inferiority because it detects proper nouns ("john" and "cincinnati") more often than the other methods. As expected, a high degree of correlation was found among the KLD, BF, and LLR, while a low degree of correlation was found between the SVM and the other methods. The BF is competitive with the SVM while statistically quantifying similarities and differences between corpora in an intuitive way. All methods characterized differences between the corpora using those clinical features that one would expect before and after surgery or before and after the date of last seizure. The BF gives insight into the accuracy of the statistical model. Here, it behaved as it should, indicating that the assumptions regarding Poisson fluctuations in the unigrams are accurate.

**Claims**

1. A computer-implemented method for the identification of epilepsy patients who are candidates for surgery by utilizing data extracted from clinical notes of an epilepsy patient to classify the patient's epilepsy as either "intractable" or "non-intractable", the method comprising:

   receiving, by a computing device, a set of data consisting of n-grams extracted from a corpus of clinical text of the epilepsy patient;
   classifying the data into one of two bins consisting of "intractable epilepsy" or "non-intractable epilepsy" by applying by a computer implemented method comprising a classifier selected from the group consisting of a pre-trained support vector machine (SVM), a log-likelihood ratio, Bayes factor, or Kullback-Leibler Divergence, wherein the classifier is trained on a training set consisting of two sets of n-grams extracted from two corpora of clinical text, a first corpus consisting of clinical text from a population of epilepsy patients that were referred for surgery and a second corpus consisting of clinical text from a population of epilepsy patients that were never referred for surgery; and
   outputting the result of the classification of the data as the classification of the patient's epilepsy as either "intractable" or "non-intractable", wherein a classification of "intractable" identifies the patient as a candidate for surgery.

2. The computer implemented method of claim 1, wherein the method further comprises extracting the n-grams from the corpus of clinical text prior to or concurrent with receiving the set of data.

3. The computer implemented method claim 2, wherein the method further comprises structuring the data prior to classifying, optionally wherein the operation of structuring the data includes one or more of tagging parts of speech, replacing abbreviations with words, correcting misspelled words, converting all words to lower-case, and removing n-grams containing non-ASCII characters.

4. The computer implemented method of any of claims 1-3, wherein the method further comprises querying a database of electronic records to identify the clinical text for inclusion in the corpus.

5. The computer implemented method of claim 1, wherein each document of the corpora of clinical text satisfies each of the following criteria: it was created for an office visit, it is over 100 characters in length, it comprises an ICD-9-CM code for epilepsy, and it is signed by an attending clinician, resident, fellow, or nurse practioner.

6. The computer implemented method of claim 1 or 5, wherein each patient of the population of patients is represented by at least 4 documents, each from a separate office visit.

7. The computer implemented method of any of claims 1-6, wherein the set of data or the training set are annotated with term classes and subclasses of an epilepsy ontology; and optionally wherein the term classes comprise one or more, or all, of the following: seizure type, etiology, epilepsy syndrome by age, epilepsy classification, treatment, and diagnostic testing.

8. The computer implemented method of any of claims 1-7, wherein the result is displayed on a graphical user interface.

9. The computer implemented method of claim 8, wherein the result comprises one or a combination of two or more of text, color, imagery, or sound.

10. The computer implemented method of any of claims 1-9, wherein the outputting further comprises sending an alert to an end-user if the results of the classification are "intractable" and the patient had a previous result of "non-intractable".

11. The computer implemented method of claim 10, wherein the alert is in the form of a visual or audio signal that is transmitted to a computing device selected from a personal computer, a tablet computer, and a smart phone, optionally wherein the alert is manifested as any of an email, a text message, a voice message, or sound.

12. The computer implemented method of claim 1, wherein the n-grams consist of unigrams, bigrams, and trigrams.

13. A clinical decision support, CDS, tool for the identification of epilepsy patients who are candidates for surgery, the CDS tool comprising a non-transitory computer readable medium storing instructions that, when executed by at least one programmable processor, cause the at least one programmable processor to perform the computer implemented method of any of claims 1-12.

14. A system comprising the CDS tool of claim 13 operatively linked to one or more databases of electronic medical records and/or clinical data.

**Patentansprüche**

1. Computerimplementiertes Verfahren für die Identifizierung von Epilepsiepatienten, die für eine Operation in Frage kommen, durch Verwenden von Daten, die aus klinischen Anmerkungen eines Epilepsiepatienten extrahiert werden, um die Epilepsie des Patienten als entweder "therapierefraktär" oder "nicht therapierefraktär" zu klassifizieren, wobei das Verfahren Folgendes umfasst:

Empfangen, durch eine Rechenvorrichtung, eines Datensatzes, der aus n-Grammen besteht, die aus einem Korpus von klinischem Text des Epilepsiepatienten extrahiert werden;
Klassifizieren der Daten in einen von zwei Bins, die aus "therapierefraktärer Epilepsie" oder "nicht therapierefraktärer Epilepsie" bestehen, durch Anwenden eines logarithmischen Verfahrens, das einen Klassifizierer umfasst, der aus der Gruppe ausgewählt ist, die aus einer vortrainierten Support-Vektor-Maschine (SVM), einem Log-Likelihood-Verhältnis, Bayes-Faktor oder Kullback-Leibler-Divergenz besteht, wobei der Klassifizierer auf einem Trainingssatz trainiert wird, der aus zwei Sätzen von n-Grammen besteht, die aus zwei Korpora von klinischem Text extrahiert werden, wobei ein erster Korpus aus klinischem Text aus einer Population von Epilepsiepatienten besteht, die für eine Operation überwiesen wurden, und ein zweiter Korpus aus klinischem Text aus einer Population von Epilepsiepatienten besteht, die nie für eine Operation überwiesen wurden; und
Ausgeben des Ergebnisses der Klassifizierung der Daten als die Klassifizierung der Epilepsie des Patienten als entweder "therapierefraktär" oder "nicht therapierefraktär", wobei eine Klassifizierung von "therapierefraktär" den Patienten als Kandidaten für eine Operation identifiziert.

2. Computerimplementiertes Verfahren nach Anspruch 1, wobei das Verfahren ferner ein Extrahieren der n-Gramme aus dem Korpus von klinischem Text vor oder gleichzeitig mit dem Empfangen des Datensatzes umfasst.

3. Computerimplementiertes Verfahren nach Anspruch 2, wobei das Verfahren ferner ein Strukturieren der Daten vor dem Klassifizieren umfasst, optional wobei der Vorgang des Strukturierens der Daten ein Markieren von Sprechteilen, Ersetzen von Abkürzungen durch Wörter, Korrigieren falsch geschriebener Wörter, Umwandeln aller Wörter in Kleinbuchstaben und/oder Entfernen von n-Grammen, die Nicht-ASCII-Zeichen enthalten, beinhaltet.

4. Computerimplementiertes Verfahren nach einem der Ansprüche 1-3, wobei das Verfahren ferner ein Abfragen einer Datenbank elektronischer Aufzeichnungen umfasst, um den klinischen Text für eine Aufnahme in den Korpus zu identifizieren.

5. Computerimplementiertes Verfahren nach Anspruch 1, wobei jedes Dokument der Korpora von klinischem Text jedes der folgenden Kriterien erfüllt: es wurde für einen Praxisbesuch erstellt, es ist über 100 Zeichen lang, es umfasst einen ICD-9-CM-Code Epilepsie und es wird von einem behandelnden Kliniker, Hausarzt, Kollegen oder Pflegefachmann unterzeichnet.

6. Computerimplementiertes Verfahren nach Anspruch 1 oder 5, wobei jeder Patient der Population von Patienten durch wenigstens 4 Dokumente repräsentiert wird, von denen jedes aus einem individuellen Praxisbesuch stammt.

7. Computerimplementiertes Verfahren nach einem der Ansprüche 1-6, wobei der Datensatz oder der Trainingssatz mit Bergriffsklassen und Unterklassen einer Epilepsieontologie kommentiert werden; und optional wobei die Begriffsklassen eine oder mehrere oder alle der Folgenden umfassen: Anfallsart, Ätiologie, Epilepsiesyndrom nach Alter, Epilepsieklassifizierung, Behandlung und diagnostische Tests.

8. Computerimplementiertes Verfahren nach einem der Ansprüche 1-7, wobei das Ergebnis auf einer grafischen Benutzeroberfläche angezeigt wird.

9. Computerimplementiertes Verfahren nach Anspruch 8, wobei das Ergebnis eines oder eine Kombination von zwei oder mehr von Text, Farbe, Bildsymbolen oder Ton umfasst.

10. Computerimplementiertes Verfahren nach einem der Ansprüche 1-9, wobei das Ausgeben ferner ein Senden einer Benachrichtigung an einen Endbenutzer umfasst, falls die Ergebnisse der Klassifizierung "therapierefraktär" sind und der Patient ein vorheriges Ergebnis "nicht therapierefraktär" aufwies.

11. Computerimplementiertes Verfahren nach Anspruch 10, wobei die Benachrichtigung in der Form eines visuellen oder Audiosignals vorliegt, das an eine Computervorrichtung übertragen wird, das aus einem Personal Computer, einem Tablet Computer und einem Smartphone ausgewählt ist, optional wobei die Benachrichtigung als eine E-Mail, eine Textnachricht, eine Sprachnachricht oder ein Ton manifestiert ist.

12. Computerimplementiertes Verfahren nach Anspruch 1, wobei die n-Gramme aus Unigrammen, Bigrammen und Trigrammen bestehen.

13. Tool für eine klinische Entscheidungsunterstützung (CDS - Clinical Decision Support) für die Identifizierung von Epilepsiepatienten, die für eine Operation in Frage kommen, wobei das CDS-Tool ein nicht flüchtiges computerlesbares Medium umfasst, das Anweisungen speichert, die, wenn sie durch wenigstens einen programmierbaren Prozessor ausgeführt werden, den wenigstens einen programmierbaren Prozessor veranlassen, das computerimplementierte Verfahren nach einem der Ansprüche 1-12 durchzuführen.

14. System, das das CDS-Tool nach Anspruch 13 umfasst, das mit einer oder mehreren Datenbanken elektronischer medizinischer Aufzeichnungen und/oder klinischer Daten wirkverknüpft ist.

**Revendications**

1. Procédé mis en œuvre par ordinateur pour l'identification de patients épileptiques qui sont candidats à la chirurgie en utilisant des données extraites de notes cliniques d'un patient épileptique pour classer l'épilepsie du patient comme « incurable » ou « non incurable », le procédé comprenant :

la réception, par un dispositif informatique, d'un ensemble de données constitué de n-grammes extraits d'un corpus de texte clinique du patient épileptique ;

la classification des données dans l'un des deux compartiments constitués d'une « épilepsie incurable » ou d'une « épilepsie non incurable » en appliquant, par un procédé mis en œuvre par ordinateur comprenant un classificateur sélectionné dans le groupe constitué d'une machine à vecteurs de support pré-entraînée (SVM), d'un logarithme du rapport de vraisemblance, du facteur de Bayes ou de la divergence de Kullback-Leibler, le classificateur étant entraîné sur un ensemble d'entraînement constitué de deux ensembles de n-grammes extraits de deux corpus de texte clinique, d'un premier corpus constitué d'un texte clinique d'une population de patients épileptiques qui ont été référés pour une intervention chirurgicale et d'un second corpus constitué d'un texte clinique provenant d'une population de patients épileptiques qui n'ont jamais été référés pour une intervention chirurgicale ; et

l'émission du résultat de la classification des données comme classification de l'épilepsie du patient comme « incurable » ou « non incurable », une classification « incurable » identifiant le patient comme un candidat à la chirurgie.

2. Procédé mis en œuvre par ordinateur selon la revendication 1, dans lequel le procédé comprend en outre l'extraction des n-grammes du corpus de texte clinique avant ou simultanément à la réception de l'ensemble de données.

3. Procédé mis en œuvre par ordinateur selon la revendication 2, dans lequel le procédé comprend en outre la structuration des données avant la classification, éventuellement l'opération de structuration des données comportant une ou plusieurs parties de balisage du discours, le remplacement des abréviations par des mots, la correction des mots mal orthographiés, la conversion de tous les mots en minuscules et la suppression des n-grammes contenant des caractères non ASCII.

4. Procédé mis en œuvre par ordinateur selon l'une quelconque des revendications 1 à 3, dans lequel le procédé comprend en outre la requête d'une base de données d'enregistrements électroniques pour identifier le texte clinique à inclure dans le corpus.

5. Procédé mis en œuvre par ordinateur selon la revendication 1, dans lequel chaque document des corpus de texte clinique satisfait à chacun des critères suivants : il a été créé pour une visite de bureau, il compte plus de 100 caractères, il comprend un code CIM-9-MC pour l'épilepsie et il est signé par un médecin clinicien traitant, un résident, un collègue ou un infirmier praticien.

6. Procédé mis en œuvre par ordinateur selon la revendication 1 ou 5, dans lequel chaque patient de la population de patients est représenté par au moins 4 documents, chacun provenant d'une visite de bureau distincte.

7. Procédé mis en œuvre par ordinateur selon l'une quelconque des revendications 1 à 6, dans lequel l'ensemble de données ou l'ensemble d'entraînement sont annotés avec des classes de termes et des sous-classes d'une ontologie d'épilepsie ; et éventuellement les classes de termes comprenant un ou plusieurs, ou tous, des éléments suivants : type de crise, étiologie, syndrome d'épilepsie par âge, classification de l'épilepsie, traitement et test de diagnostic.

8. Procédé mis en œuvre par ordinateur selon l'une quelconque des revendications 1 à 7, dans lequel le résultat est affiché sur une interface utilisateur graphique.

9. Procédé mis en œuvre par ordinateur selon la revendication 8, dans lequel le résultat comprend une ou plusieurs combinaisons de deux ou plusieurs éléments de texte, de couleur, d'imagerie ou de son.

10. Procédé mis en œuvre par ordinateur selon l'une quelconque des revendications 1 à 9, dans lequel l'émission comprend en outre l'envoi d'une alerte à un utilisateur final si les résultats de la classification sont « incurables » et que le patient avait un résultat antérieur « non incurable ».

11. Procédé mis en œuvre par ordinateur selon la revendication 10, dans lequel l'alerte se présente sous la forme d'un signal visuel ou audio qui est transmis à un dispositif informatique sélectionné à partir d'un ordinateur personnel, d'une tablette et d'un téléphone intelligent, éventuellement l'alerte se manifestant par un e-mail ou un message texte ou un message vocal ou un son.

12. Procédé mis en œuvre par ordinateur selon la revendication 1, dans lequel les n-grammes sont constitués d'unigrammes, de bigrammes et de trigrammes.

13. Outil de support de décision clinique, CDS, pour l'identification de patients épileptiques qui sont candidats à la chirurgie, l'outil CDS comprenant un support non transitoire lisible par ordinateur stockant des instructions qui, lorsqu'elles sont exécutées par au moins un processeur programmable, amènent l'au moins un processeur programmable à effectuer le procédé mis en œuvre par ordinateur selon l'une quelconque des revendications 1 à 12.

14. Système comprenant l'outil CDS selon la revendication 13, lié fonctionnellement à une ou plusieurs bases de données de dossiers médicaux électroniques et/ou de données cliniques.

Surgery

Intractable
Patient

Surgery
Intake From

1st Tx        2nd Tx

Time

1st Tx        2nd Tx

Date of
Last Seizure

Seizure
Freedom

Non-intractable
Patient

# FIG. 1

Language of Intractable

e e e

e e e

e e e<sup>e</sup> e e<sup>e</sup>

Language of Non-Intractable

Year 1          Year 4          Year 6          Year 10

Language of Intractable

e

e e<sup>e</sup>

e e

e e e<sup>e</sup> e e<sup>e</sup>

Language of Non-Intractable

Year 1          Year 4          Year 6          Year 10

# FIG. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2013035961 A1

**Non-patent literature cited in the description**

- **KWAN et al.** *NEJ Med.,* 2000, vol. 342 (5), 314-319
- **KWAN et al.** *Epilepsia,* 2010, vol. 51 (6), 1069-1077
- **HIMES et al.** *J. Am. Med. Inform. Assoc.,* 2009, vol. 16 (3), 371-379
- **HAUG, P.J.** *J. Am. Med. Inform. Assoc.,* 2013, e102-e110
- **VERSPOOR et al.** *BMC Bioinfo,* 2009, vol. 10 (1
- **CATICHA ; GIFFIN.** *AIP Conf. Proc.,* 2006, vol. 872, 31
- **COLLINS ; LIU ; LEORDEANU.** *IEEE Transactions,* 2005, vol. 27 (10), 1631-1643
- **GUYON ; WESTON ; BARNHILL ; VAPNIK.** *Machine Learning,* 2002, vol. 46 (1-3), 389-422